**Europäisches Patentamt**

**European Patent Office**

**Office européen des brevets**

(19)

(11) Veröffentlichungsnummer : **0 010 271**
**B1**

(12) **EUROPÄISCHE PATENTSCHRIFT**

(45) Veröffentlichungstag der Patentschrift :
07.04.82

(51) Int. Cl.³ : **C 07 D201/16, C 07 D223/10**

(21) Anmeldenummer : **79103938.1**

(22) Anmeldetag : **12.10.79**

(54) **Kristallisationsverfahren zur Reinigung von Rohcaprolactam.**

(30) Priorität : **17.10.78 DE 2845075**

(43) Veröffentlichungstag der Anmeldung :
**30.04.80 (Patentblatt 80/09)**

(45) Bekanntmachung des Hinweises auf die Patenterteilung : **07.04.82 Patentblatt 82/14**

(84) Benannte Vertragsstaaten :
**BE CH DE GB IT NL**

(56) Entgegenhaltungen :
AT - B - 311 376
AT - B - 318 640
CH - A - 557 353
CH - A - 564 528
CH - A - 581 102
DD - A - 91 035
DD - A - 132 491
DD - A - 133 092
DE - A1 - 2 641 478
GB - A - 1 403 341

(73) Patentinhaber : **BASF Aktiengesellschaft
Carl-Bosch-Strasse 38
D-6700 Ludwigshafen (DE)**

(72) Erfinder : **Horn, Peter, Dr. Dipl.-Chem.
Im Bruennel 20
D-6945 Hirschberg (DE)**
Erfinder : **Fuchs, Hugo, Dr. Dipl.-Chem.
Egellstrasse 28
D-6700 Ludwigshafen (DE)**

## Kristallisationsverfahren zur Reinigung von Rohcaprolactam

Gegenstand der Erfindung ist ein Kristallisationsverfahren zur Reinigung von Rohcaprolactam, das durch Umlagerung von Cyclohexanonoxim in der Gasphase an Bortrioxid enthaltenden Katalysatoren erhalten wurde.

Caprolactam, das durch Umlagerung in der Gasphase an Bortrioxid enthaltenden Katalysatoren hergestellt wurde, enthält als Verunreinigungen neben 0,05 bis 5 Mol-% Borsäure noch eine Reihe von Verunreinigungen, deren chemische Zusammensetzung nicht bekannt ist. Diese Verunreinigungen zeichnen sich durch einen unangenehmen Geruch aus und verleihen dem Caprolactam eine hohe Absorption in ultravioletten Bereich. Beide Eigenschaften sind unerwünscht. Entsprechend der DE-OS 25 50 934 soll katalytisch hergestelltes Caprolactam gereinigt werden, durch Behandeln mit einer Base in Gegenwart von Toluol und Wasser. Hierbei erhält man 3 Schichten, die von einander getrennt werden müssen. Abgesehen davon, daß hieraus nicht zu entnehmen ist wie die UV-Zahl gesenkt werden kann, ist die Trennung von 3 Phasen technisch sehr aufwendig. Ferner ist aus der DE-OS 26 41 478 ein Verfahren zur Reinigung von Caprolactam aus der Gasphasenumlagerung bekannt, bei dem man das Rohlactam in Anwesenheit von Wasser mit Kaliumpermanganat behandelt und anschließend aus einem Lösungsmittel umkristallisiert. Die Behandlung mit Kaliumpermanganat ist in sofern aufwendig, als das ausgeschiedene Mangandioxid nicht leicht abzutrennen ist.

Entsprechend der GB-PS 1 403 341 sollen für die Umkristallisation von Caprolactam aus der Gasphasenumlagerung spezifische Lösungsmittel, insbesondere Dimethylformamid, Dimethylacetamid, Ethylformiat, Tetrahydrofuran oder Dioxan verwendet werden, während Alkanole als weniger wirkungsvoll dargestellt werden. Es ist jedoch nicht zu entnehmen, welche Maßnahmen zu ergreifen sind, um die UV-Kennzahl von rohem Caprolactam auf das gewünschte Maß zu senken. Vielmehr wird ein Weg gewiesen, Lösungsmittel anderer Verbindungsklassen als Alkanole zu untersuchen, um eine verbesserte Reinigung zu erzielen.

Es war deshalb die technische Aufgabe gestellt, ein einfaches, technisch nicht aufwendiges Verfahren zur Verfügung zu stellen, bei dem es gelingt, die Verunreinigungen auszuscheiden, die für den unangenehmen Geruch und die hohe UV-Zahl verantwortliche sind. Ferner soll gleichzeitig die enthaltene Borsäure abgetrennt werden.

Diese technische Aufgabe wir gelöst in einem Kristallisationsverfahren zur Reinigung von Rohcaprolactam, das durch Umlagerung von Cyclohexanonoxim in der Gasphase an Bortrioxid enthaltenen Katalysatoren erhalten wurde, wobei man aus einer Schmelze, die je 100 Gewichtsteile Rohcaprolactam, 5 bis 30 Gewichtsteile primäre Alkanole oder Fettsäuren, die eine Kohlenstoffkette mit 6 bis 10 Kohlenstoffatomen haben, und Alkylreste mit 1 bis 5 Kohlenstoffatomen als Substituenten aufweisen sowie 0 bis 5 Gewichtsteile Wasser enthält, durch Abkühlen Caprolactam auskristallisiert und von der Mutterlauge trennt.

Das neue Verfahren hat den Vorteil, daß es technisch einfach durchführbar ist, und Verunreinigungen, die für den unangenehmen Geruch und die hohe UV-Zahl verantwortlich sind, auf einfache Weise wirksam entfernt werden.

Rohcaprolactam, das als Ausgangsstoff für die erfindungsgemäße Reinigung verwendet wird, erhält man beispielsweise nach dem in der DE-AS 10 55 537 beschriebenen Verfahren durch Abscheiden von Caprolactam aus solchen enthaltenden Dämpfen in einer Kolonne mit Wasser im Gegenstrom. Für die Reinigungsoperation geht man von einer Schmelze aus, die je 100 Gewichtsteile Rohcaprolactam 5 bis 30 Gewichtsteile primäre Alkanole oder Fettsäuren, die eine Kohlenstoffkette mit 6 bis 10 Kohlenstoffatomen haben und Alkylreste mit 1 bis 5 Kohlenstoffatomen als Substituenten aufweisen, enthält. Geeignete Verbindungen sind beispielsweise 2-Ethylhexanol-(1), Isononanol oder Isodecanol, ferner 2-Ethylhexansäure. Besonders bewährt haben sich primäre Alkanole der genannten Kohlenstoffzahl, insbesondere 2-Ethylhexanol-(1). Ferner enthält die Schmelze 0 bis 5, insbesondere 1 bis 4 Gewichtsteile Wasser je 100 Gewichtsteile Rohcaprolactam. Die Schmelze wird beispielsweise hergestellt, indem man zu geschmolzenem Rohcaprolactam die genannten Komponenten zugibt oder festes Caprolactam zusammen mit den genannten Komponenten aufschmilzt. Eine solche Schmelze aus 100 Gewichtsteilen Rohcaprolactam, 17,6 Gewichtsteilen 2-Ethylhexanol-(1) und 3,1 Gewichtsteilen Wasser erstarrt beispeilsweise bei 55 °C.

Aus der oben beschriebenen Schmelze wird durch Abkühlen Caprolactam auskristallisiert. Vorteilhaft kühlt man auf eine Temperatur von 10 bis 30 °C, insbesondere von 15 bis 25 °C ab. Die Kristallisation wird in Vorrichtungen, die für die Kristallisation in der Technik bekannt sind, wie sie beispielsweise in « Grundoperation Chemischer Verfahrenstechnik » von W.R.A. Vauk und H.A. Müller, Verlag Theodor Steinkopff, Dresden und Leipzig, 1966, 2. Auflage, Seiten 528 bis 535 in dem Kapitel « Kristallisatoren » beschrieben sind, durchgeführt. Das auskristallisierte Caprolactam wird dann von der Mutterlauge abgetrennt, z.B. durch Dekantieren oder insbesondere durch Abschleudern.

Je nach dem Gehalt an Verunreinigungen reicht eine einmalige Reinigungsoperation oder falls erforderlich kann die erfindungsgemäße Reinigungsoperation mehrmals wiederholt werden. Zweckmäßig wird aus der Mutterlauge das darin enthaltene Caprolactam durch Destillation zurückgewonnen und wieder dem Rohcaprolactam zugeführt. Die ebenfalls zurückgewonnenen Alkanole und Carbonsäuren werden ebenfalls wieder für die Reinigungsoperation verwendet. Die Verunreinigungen werden als Rückstand aufgeschleust.

Das nach dem Verfahren der Erfindung gereinigte Caprolactam wird vor seiner Weiterverwendung zweckmäßiger noch einmal destilliert.

Caprolactam eignet sich zur Herstellung von Polyamiden. Das Verfahren nach der Erfindung sei an folgenden Beispielen veranschaulicht.

Beispiel 1

Eine Schmelze aus
100 Gewichtsteilen Rohcaprolactam mit einer UV-Kennzahl 16 000
17,6 Gewichtsteilen 2-Ethylhexanol-(1) und
3,1 Gewichtsteilen Wasser
wird unter Rühren auf 20 °C abgekühlt und die Maische zentrifugiert. Das kristallisierte Caprolactam (73,6 Gewichtsteile) hat eine UV-Zahl von 1 758. Eine Schmelze dieses Caprolactams mit 17,6 Gewichtsteilen 2-Ethylhexanol-(1) wird nochmals kristallisiert und zentrifugiert. Als Kristallisat erhält man 56,5 Gewichtsteile Caprolactam mit einer UV-Zahl von 132. Aus den Mutterlaugen erhält man durch Destillation 34 Gewichtsteile 2-Ethylhexanol, 37 Gewichtsteile Caprolactam (UV-Zahl 7 100), 3,1 Gewichtsteile Wasser und 6,2 Gewichtsteile Rückstand.

Beispiel 2

Eine Schmelze aus
65 Gewichtsteilen Rohcaprolactam mit einer UV-Zahl von 16 000
31 Gewichtsteilen Caprolactam aus der Mutterlauge von Beispiel 1 (UV-Zahl 7 100)
17,6 Gewichtsteilen 2-Ethylhexanol-(1) und
3,1 Gewichtsteilen Wasser
wird unter Rühren auf 20 °C abgekühlt und kristallines Caprolactam aus der Maische abzentrifugiert. Das kristallisierte Caprolactam (70,7 Gewichtsteile) hat eine UV-Zahl von 1 287. Eine Schmelze dieses Caprolactams (70 Gewichtsteile) mit 17,6 Gewichtsteilen 2-Ethylhexanol wird nochmals kristallisiert und zentrifugiert. Als Kristallisat erhält man Caprolactam (51,2 Gewichtsteile) mit einer UV-Zahl von 70. Durch Destillieren erhält man daraus Caprolactam folgender Qualität :

| | |
|---|---|
| Erstarrungspunkt (°C) | 69,05 |
| ph | 7,13 |
| Gehalt an flüchtigen Basen | 0,38 mÄq/kg |
| PAZ | 8,6 |
| UV-Zahl | 11,5 |

PAZ = Permanganat - Absorptionszahl

Gemessen wird die Extinktion aus der Lichtdurchlässigkeit einer 1-prozentigen Caprolactamlösung in Wasser (50 ml bzw. 100 ml Lösung) nach Zugabe von 0,01 n $KMnO_4$-Lösung (1 bzw. 2 ml) bei 25 °C gegen eine gleiche Lösung ohne Caprolactam nach 600 sec.

UV-Zahl

Prinzip : Im Spektralbereich von 360 bis 270 nm wird die Absorption des Caprolactams gemessen und nach Umsetzung in einer Kennzahl ausgedrückt.

Analysengeräte : 1 registrierendes Einstrahlspektralphotometer (Carl Zeiss DMR/21), 1 Erlenmeyerkolben (200 ml), 2 Quarz-Küvetten mit Deckel 10 cm lang (Schichtdicke 10 cm).

Vorschrift : 50 mg Caprolactam werden in einem Erlenmeyerkolben in 50 g bidestilliertem Wasser kalt aufgelöst. Mit dieser Lösung wird eine Küvette bis zur Eichmarke gefüllt. Die zweite Küvette wird mit dem gleichen bidestillierten Wasser gefüllt und stellt die Vergleichslösung dar.

Nun werden beide Küvetten mit den Deckeln verschlossen, die geschliffenen Flächen mit Seidenpapier gereinigt und in die Küvettenhalter eingesetzt. Dann wird gemäß Geräteanleitung das Spektrum zwischen 370 nm und 260 nm aufgenommen. Die Registriergeschwindigkeit beträgt 50. Die Extinktionsmessung wird im Meßbereich 0-1 ausgeführt.

Ist die Aufnahme beendet, wird von 270 bis 360 nm alle 10 nm eine Markierung auf dem Papier angebracht.

Auswertung : Aus dem Diagram werden die Extinktionen bei 270, 280, 290, 300, 310, 320, 330, 340, 350 und 360 nm abgelesen und addiert.

Die Summe der 10 Extinktionswerte wird mit 2 multipliziert und ergibt die UV-Kennzahl. Die UV-Kennzahl wird also immer auf 100 %iges Caprolactam und auf eine Schichtdicke von 10 cm bezogen.

**Ansprüche**

1. Kristallisationsverfahren zur Reinigung von rohem Caprolactam, das durch Umlagerung von Cyclohexanonoxim in der Gasphase an Bortrioxid enthaltenden Katalysatoren erhalten wurde, dadurch gekennzeichnet, daß man aus einer Schmelze, die je 100 Gewichtsteile Rohcaprolactam, 5 bis 30 Gewichtsteile primäre Alkanole oder Fettsäuren, die eine Kohlenstoffkette mit 6 bis 10 Kohlenstoffatomen haben und Alkylreste mit 1 bis 5 Kohlenstoffatomen als Substituenten aufweisen, sowie 0 bis 5 Gewichtsteile Wasser enthält, durch Abkühlen Caprolactam auskristallisiert und von der Mutterlauge trennt.

2. Kristallisationsverfahren nach Anspruch 1, dadurch gekennzeichnet, daß man die Schmelze auf 10 bis 30 °C abkühlt.

3. Kristallisationsverfahren nach den Ansprüchen 1 und 2, dadurch gekennzeichnet, daß man 2-Ethylhexanol-(1) verwendet.

**Claims**

1. A crystallization process for purifying crude caprolactam which has been obtained by rearrangement of cyclohexanone-oxime in the gas phase over a catalyst containing boron trioxide, characterized in that caprolactam is crystallized out, by cooling, from a melt which contains, per 100 parts by weight of crude caprolactam, from 5 to 30 parts by weight of primary alkanols or fatty acids which have a carbon chain of 6 to 10 carbon atoms and are substituted by alkyl radicals of 1 to 5 carbon atoms, and from 0 to 5 parts by weight of water, and is separated from the mother liquor.

2. A crystallization process as claimed in claim 1, characterized in that the melt is cooled to from 10 to 30 °C.

3. A crystallization process as claimed in claims 1 and 2, characterized in that 2-ethylhexan-1-ol is used.

**Revendications**

1. Procédé de purification par cristallisation de la caprolactame brute, obtenue par transposition de l'oxime de la cyclohexanone en phase gazeuse en présence de catalyseurs contenant du trioxyde, de bore, caractérisé en ce que l'on provoque par refroidissement d'une masse fondue qui contient, pour 100 parties en poids de caprolactame brute, 5 à 30 parties en poids d'alcanols primaires ou d'acides gras, qui possèdent une chaîne carbonée avec 6 à 10 atomes de carbone et sont substitués par des radicaux alcoyle en $C_1$ à $C_5$, ainsi que 0 à 5 parties en poids d'eau, la cristallisation de la caprolactame, suivie de la séparation de celle-ci de la lessive mère.

2. Procédé de purification par cristallisation suivant la revendication 1, caractérisé en ce que la masse fondue est refroidie entre 10 et 30 °C.

3. Procédé de purification par cristallisation suivant l'une des revendications 1 et 2, caractérisé en ce que l'on emploie l'éthyl-2 hexanol-(1).